(19) Europäisches Patentamt
European Patent Office
Office européen des brevets

(11) **EP 2 606 931 B1**

## (12) EUROPEAN PATENT SPECIFICATION

(45) Date of publication and mention
of the grant of the patent:
**16.12.2015 Bulletin 2015/51**

(51) Int Cl.:
**A61N 1/36** *(2006.01)*      **A61B 5/1455** *(2006.01)*
**A61B 5/16** *(2006.01)*      **A61H 39/00** *(2006.01)*
**A61B 5/00** *(2006.01)*      **A61M 21/00** *(2006.01)*
**A61N 1/04** *(2006.01)*

(21) Application number: **11195632.2**

(22) Date of filing: **23.12.2011**

(54) **Brain state support apparatus and program**

Hirnzustand-Hilfsvorrichtung und Programm

Appareil de support d'état cérébral et programme

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.06.2013 Bulletin 2013/26**

(73) Proprietor: **Minami, Mitsunori
Sakai-shi, Fukui-ken 910-0273 (JP)**

(72) Inventors:
• **Minami, Mitsunori
Fukui-ken
910-0273 (JP)**
• **Kato, Toshinori
Tokyo 108-0071 (JP)**

(74) Representative: **Zimmermann & Partner
Patentanwälte mbB
Postfach 330 920
80069 München (DE)**

(56) References cited:
JP-A- 2002 177 282      US-A1- 2007 167 690
US-A1- 2008 269 832      US-A1- 2008 288 016
US-A1- 2009 228 084      US-A1- 2010 137 937

• LITSCHER GERHARD: "Bioengineering
assessment of acupuncture, part 5: cerebral
near-infrared spectroscopy", CRITICAL
REVIEWS IN BIOMEDICAL ENGINEERING, CRC
PRESS, BOCA RATON, FL, US, vol. 34, no. 6, 1
January 2006 (2006-01-01), pages 439-457,
XP009159146, ISSN: 0278-940X
• AKIKAZU SAKUDO ET AL: "Visible and near-
infrared spectral changes in the thumb of patients
with chronic fatigue syndrome", CLINICA
CHIMICA ACTA, vol. 403, no. 1-2, 1 May 2009
(2009-05-01) , pages 163-166, XP55027420, ISSN:
0009-8981, DOI: 10.1016/j.cca.2009.02.010
• GERHARD LITSCHER ET AL: "Near-infrared
spectroscopy for objectifying cerebral effects of
needle and laserneedle acupuncture",
SPECTROSCOPY, vol. 16, no. 3-4, 1 January 2002
(2002-01-01), pages 335-342, XP55026433, DOI:
10.1155/2002/863467

EP 2 606 931 B1

**Description**

[Technical Field]

The present invention relates to a brain state support apparatus and program for supporting the brain state of the human body and particularly to a brain state support apparatus and program for supporting the brain state such that it can be maintained at a relaxation mode and an intensive mode or shifted to the other states using the near-infrared spectroscopy (NIRS).

[Background Art]

In the past, various means such as massage, acupuncture and sleeping have been adopted to relax the brain state or to increase the power of concentration. (This technique will be referred to "the prior art 1" later.)

Patent document 1 (Japanese Laid-Open Patent Application 2002-177282) discloses a method of measuring changed concentrations in oxyhemoglobin and deoxyhemoglobin using the near-infrared spectroscopy before an external stimulus to be evaluated is applied to a test subject and after it has been applied to the same test subject, then hearing the subjective amenity from the test subject when said external stimulus is applied thereto, and estimating the appropriateness of the external stimulus to the human body based on the measurements and the subjective amenity.

The near-infrared spectroscopy means a method of irradiating a brain with a feeble near-infrared light (e.g., 680-1300 nanometers) through the skull and scalp of a human body and measuring the changed concentrations of oxyhemoglobin (Oxy-H b; $HbO_2$) and deoxyhemoglobin (Deoxy-Hb; Hb) in the blood in the brain surface immediately inside the brain (cerebral cortex). (This method will be referred to "the prior art 2" later.)

Further, a technique for assessing cortical activation in the brain due to acupuncture is described in LITSCHER GERHARD: "Bioengineering assessment of acupuncture, part 5: cerebral near-infrared spectroscopy", CRITICAL REVIEWS IN BIOMEDICAL ENGINEERING vol. 34, no. 6, 1 January 2006, pages 439-457.

Further, spectroscopic differences in the thumb of patients having chronic fatigue syndrome were analyzed in AKIKAZU SAKUDO ET AL: "Visible and near-infrared spectral changes in the thumb of patients with chronic fatigue syndrome", CLINICA CHIMICA ACTA, vol. 403, no. 1-2, 1 May 2009, pages 163-166.

Further, US 2007/167690 A1 describes a mind-body correlation data evaluation apparatus which includes a stimulation providing section, a physiological response data collecting section, a subjective rating and action rating data collecting section, a control and calculating section and a data feed-back section. The control and calculating section evaluates a mind-body state of the subject based on the stimulation, and at least one of the physiological response data, the subjective rating data, and the action rating data. The data feed-back section feeds back to the subject at least the mind-body state of the subject.

US 2009/228084 A1 describes a method of non invasive stimulation of acupuncture utilizing at least one electrostatic field.

[Summary of the Invention]

[Problems to be solved by the Invention]

The prior art 1 raises a problem in that it cannot be objectively known how brain state is actually influenced by massage, acupuncture, sleep.

Further, the prior art 1 raises another problem in that after having received massage and acupuncture, the effectiveness will be disappeared without continuation as soon as the brain is tensed or directed to another thing.

With the acupuncture, a beginner might have a resistance for damaging a body.

On the other hand, the prior art 2 can objectively know what kind of state the brain state is actually in. However, the prior art 2 raises a problem in that the brain state cannot be maintained at the same state or supported to shift the brain state from one to another.

Further, it is difficult that the prior art 2 judges the brain state precisely since it does not use an oxygen saturation (exchange) index to determine the cerebral oxygen consumption.

The present invention is made to solve the above problems at least partially, its object being to provide a brain state support apparatus and program which can objectively know what kind of state a brain state is in and which can support the brain state for being maintained at the same state and for shifting the brain state from one to another.

[Means to solve the Problems]

The present invention provides a brain state support apparatus according to claim 1, a program according to claim 8 and a computer-readable medium according to claim 11.

According to an aspect of the invention, a brain state support apparatus is characterized in that it comprises:

stimulus applying means for stimulating an acupoint in (or of) the human body with an electrical signal of a predetermined frequency;

light detection means comprising a light emitting section for irradiating the human body with a light at a predetermined region, and a light receiving section for receiving and sensing a light emitted from the interior of the human body (or a light from the interior of the human body); and

a main apparatus body for controlling the stimulus applying means and the light detection means,

said main apparatus body comprising:

calculating means for calculating the changed amount of total hemoglobin that is the sum of the changed amount of oxyhemoglobin plus the changed amount of deoxyhemoglobin and the changed amount of oxygen saturation that is a difference between the changed amount of the oxyhemoglobin and the changed amount of the deoxyhemoglobin, based on light information detected by the light detecting means before and after stimulating the acupoint;

determination means for applying a stimulus of an electrical signal having a predetermined frequency to the acupoint of the human body to determine whether the brain state of the human body is in at least one of a relaxation mode, a concentration mode and an intermediate mode (whether the mode of the brain state is at least one of these modes), based on the changed amount of total hemoglobin and the changed amount of oxygen saturation change which are calculated by the calculating means; and

stimulus adjusting means for adjusting the quantity of stimulus which is applied to the acupoint of the human body with the electrical signal having a predetermined frequency by the stimulus applying means such that the mode of the brain state determined by the determination means can be maintained or shifted to the other mode of the brain state (such as to selectively maintain the mode of the brain state determined by the determination means or shift it to another one of the modes of the brain state).

For example, the acupoint of the human body stimulated by the stimulus applying means is in the region of left thumb.

For example, the region detected by the light detecting means is the cerebral frontal lobe.

The determination means may be configured to determine that the brain state is in the relaxation mode when the changed amount of total hemoglobin is increased and also the changed amount of oxygen saturation is increased by stimulating the acupoint of the human body with an electrical signal of a first frequency; to determine that the brain state is in the concentration mode when the changed amount of total hemoglobin is decreased and also the changed amount of oxygen saturation is decreased by stimulating the acupoint of the human body with an electrical signal of a second frequency; and to determine that the brain state is in the intermediate mode in the other cases.

The stimulus adjusting means are configured to stimulate the acupoint of the human body with the electrical signal of the first frequency at (for) a predetermined interval of time when it is wanted to maintain the brain state at the relaxation mode or to shift it to the relaxation mode; and to stimulate the acupoint of the human body with the electrical signal of the second frequency at (for) a predetermined interval of time when (if) it is wanted to maintain the brain state at the concentration mode or to shift it to the concentration mode.

For example, the first frequency is 3Hz and the second frequency is 10Hz.

The stimulus adjusting means may be configured to increase the quantity of stimulus when the brain state is to be shifted to the other modes rather than when the brain state is to be maintained at the same mode.

The present invention also provides a program according to claim 8, causing a process to perform in the main apparatus body of the brain state support apparatus.

[Advantages of the Invention]

According to the present invention, the following advantages are provided:

(1) The desired brain state can be provided simply and easily without massage, acupuncture or sleep.
(2) The brain state can be objectively known.

Additionally, the brain state can be maintained in the same state and can be shift to the other states. When a person is tired, has many idle thoughts or is irritated with many idle thoughts or not placed in concentration, for example, the present invention can place the brain state in the concentration mode to increase the power of concentration.

When a person have a work and if the present invention determines that the person is in the relaxation mode, the present invention can forcibly shift his or her brain state to the concentration mode.

On the contrary, the present invention can shift the brain state from the concentration mode to the relaxation mode.

According to the present invention, still further, an intensified concentration state or a relaxation state can be prolonged.

[Brief Description of the Drawings]

[Fig. 1]

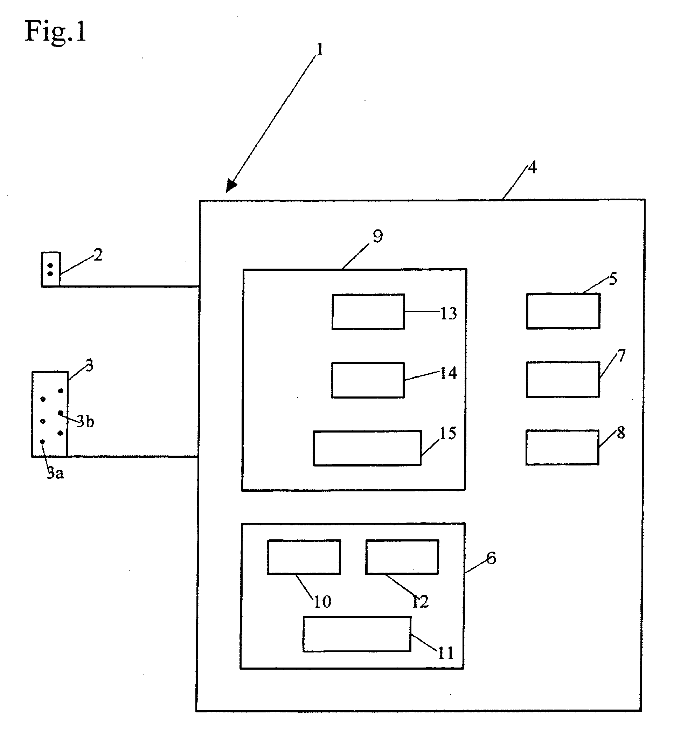

Fig. 1 is a block diagram illustrating the structure of a brain state support apparatus according to one embodiment of the present invention.
[Fig. 2]

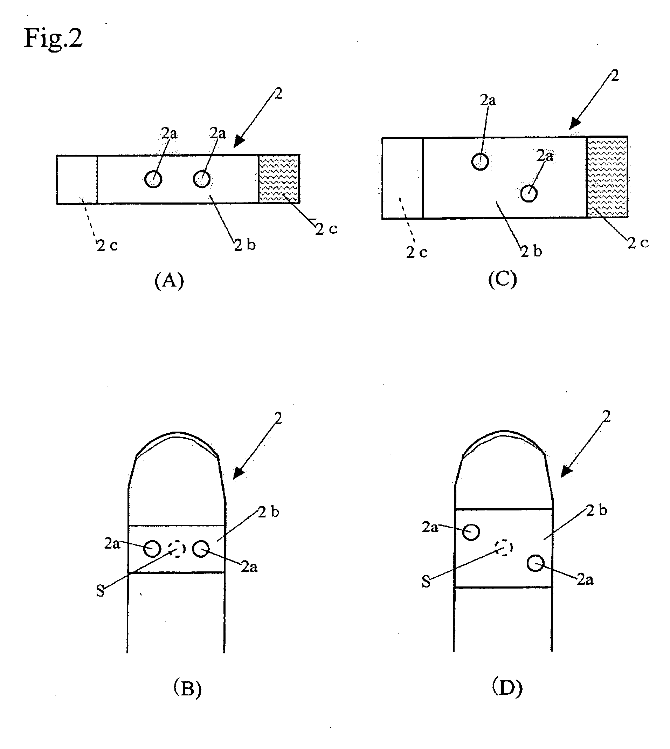

Fig. 2 (A) is a front view showing a stimulus applying device;
Fig. 2 (B) illustrates the stimulus applying device attached to a thumb;
Fig. 2 (C) is a front view illustrating a stimulus applying device according to another embodiment of the present invention; and
Fig. 2 (D) illustrates the stimulus applying device of Fig. 2 (C) attached to a thumb.

[Fig. 3]

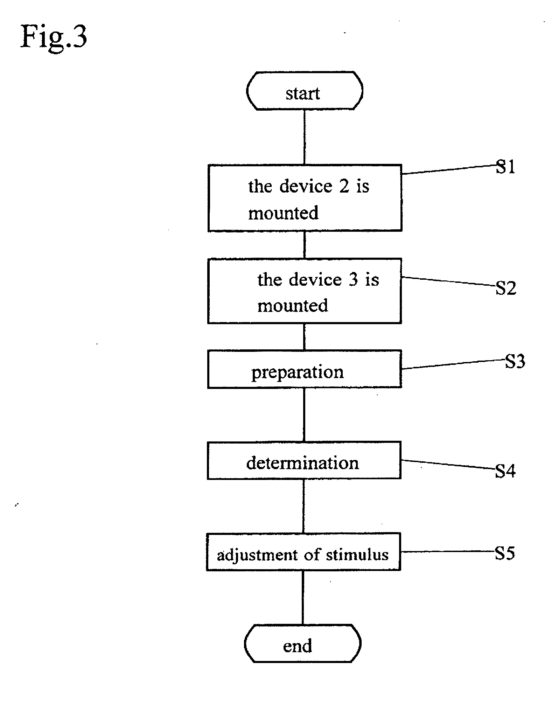

Fig. 3 is a flowchart illustrating the operation of a brain state support apparatus according to one embodiment of the present invention.
[Fig. 4]

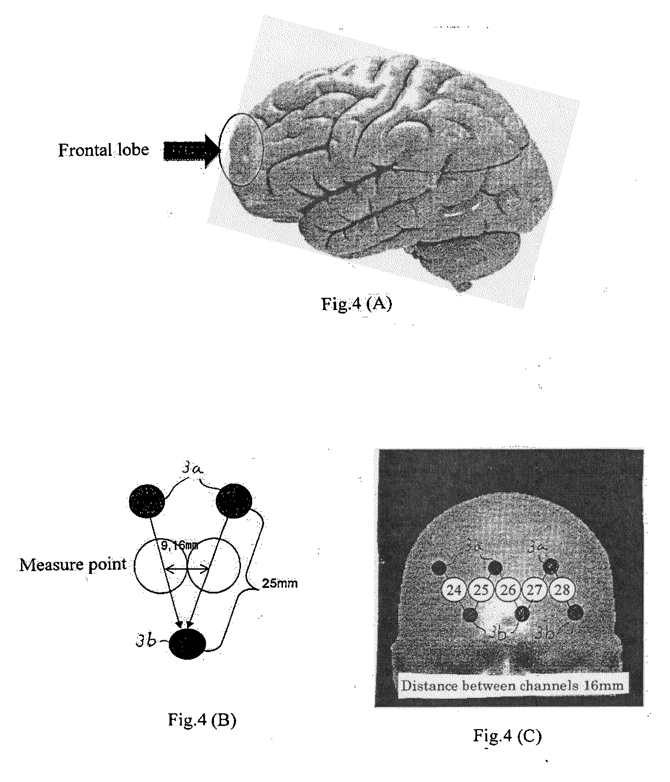

Figs. 4 (A) - (C) illustrate a light detecting device.
[Fig. 5]

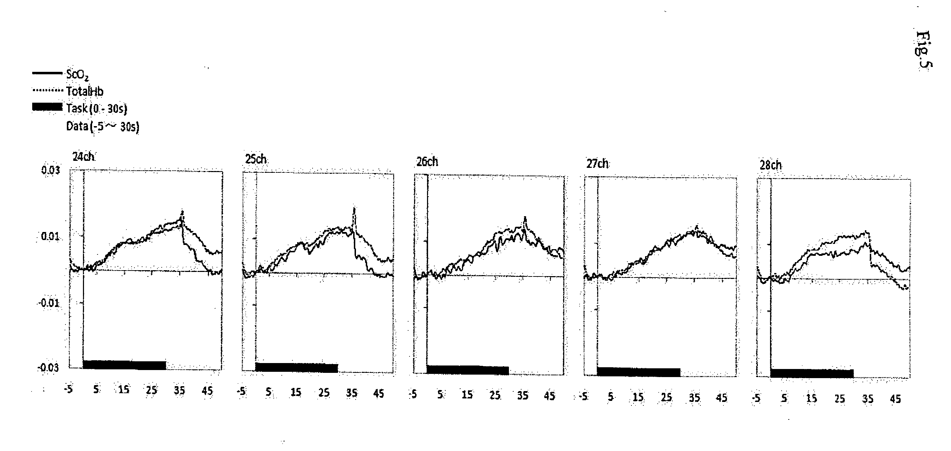

Fig. 5 shows graphs showing changes in the changed amount of total hemoglobin and the changed amount of oxygen saturation in the frontal lobe when an electrical stimulus of 3Hz is applied to a region adjacent to the center of the side of the hand palm between the first and second joints of the left hand thumb.
[Fig. 6]

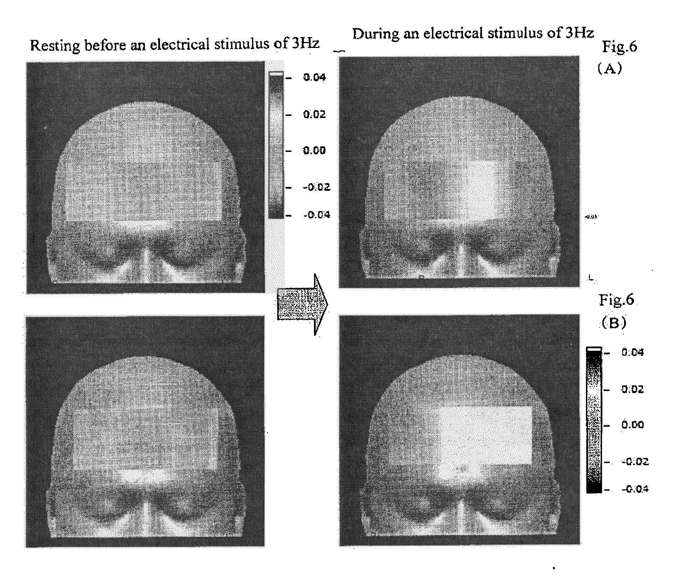

Fig. 6 (A) visually illustrates a change in the changed amount of total hemoglobin in the frontal lobe on resting before an electrical stimulus of 3Hz is applied to a region adjacent to the center of the side of the hand palm between the first and second joints of the left hand thumb and on stimulation after such an electrical stimulus has been applied to the same region.
Fig. 6 (B) visually illustrates a change in the changed amount of oxygen saturation.

[Fig. 7]

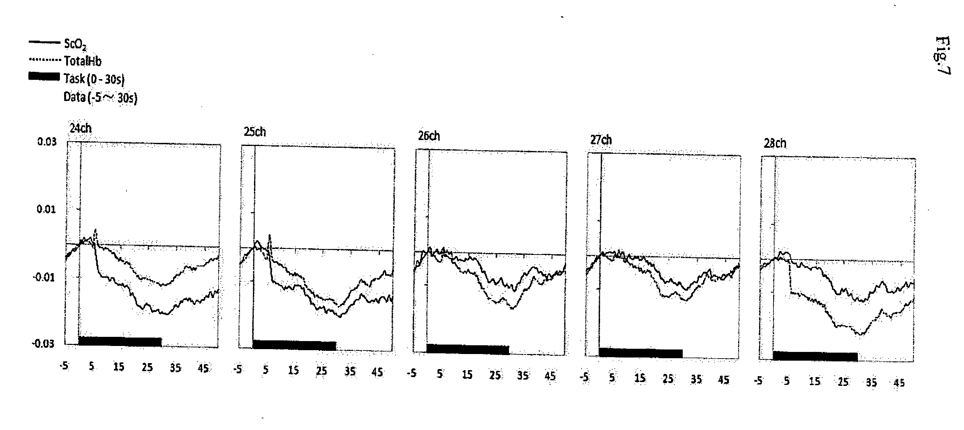

Fig. 7 shows graphs illustrating changes in the changed amount of total hemoglobin and the changed amount of oxygen saturation in the frontal lobe when an electrical stimulus of 10Hz is applied to a region adjacent to the center of the side of the hand palm between the first and second joints of the left hand thumb.
[Fig. 8]

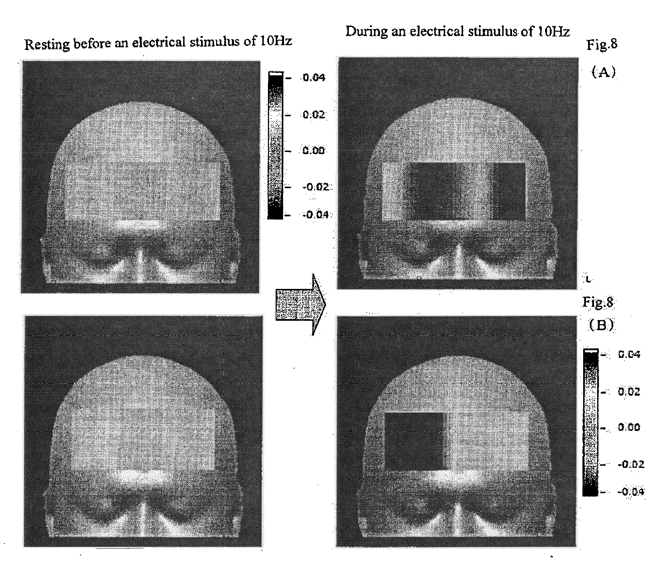

Fig. 8 (A) visually illustrates a change of the changed amount of total hemoglobin in the frontal lobe on resting before an electrical stimulus of 10Hz is applied to a region adjacent to the center of the side of the hand palm between the first and second joints of the left hand thumb and on stimulation after such an electrical stimulus has been applied to the same region.
Fig. 8 (B) visually illustrates a change in the changed amount of oxygen saturation.

[Fig. 9]

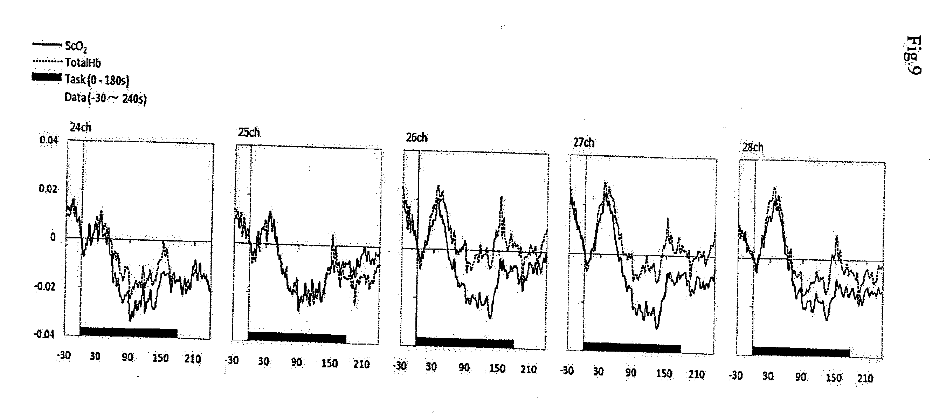

Fig. 9 shows graphs showing changes in the changed amount of total hemoglobin and the changed amount of oxygen saturation in the frontal lobe when an acupuncture stimulus is applied to a region adjacent to the center of the side of the hand palm between the first and second joints of the left hand thumb.
[Fig. 10]

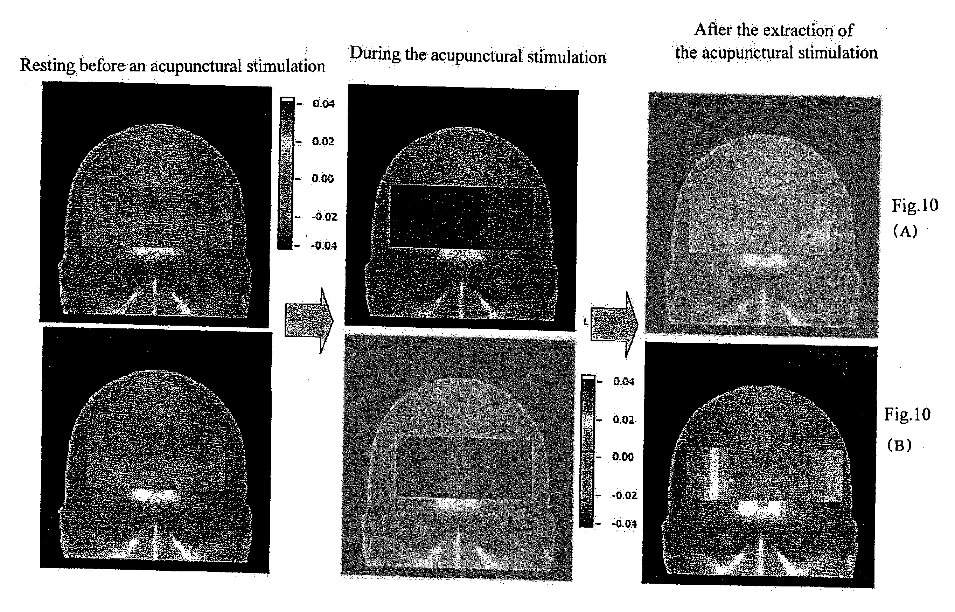

Fig. 10 (A) visually illustrates changes in the changed amount of total hemoglobin in the frontal lobe on resting when an acupuncture stimulus is applied to a region adjacent to the center of the side of the hand palm between the first and second joints of the left hand thumb, during the acupunctural stimulation and after the extraction of the acupunctural stimulation.

Fig. 10 (B) visually illustrates a change in the changed amount of oxygen saturation.

[Fig. 11]

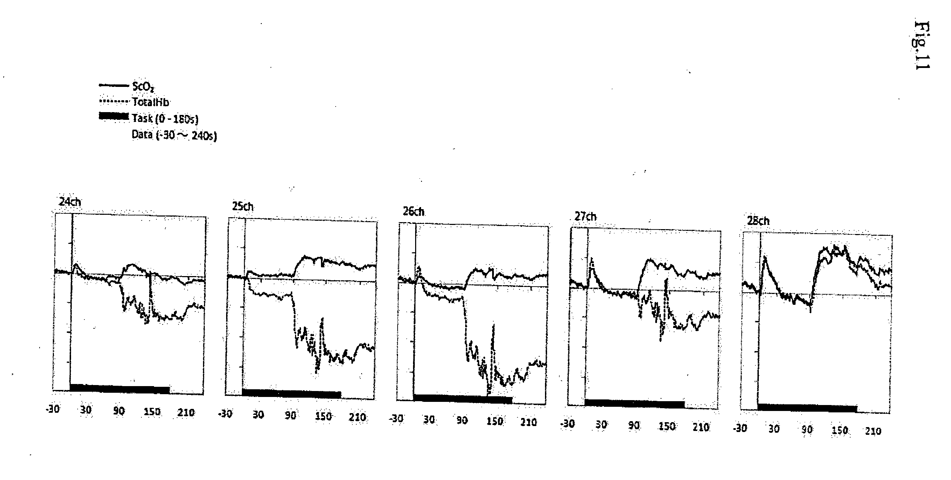

Fig. 11 shows graphs illustrating changes in the changed amount of total hemoglobin and the changed amount of oxygen saturation in the frontal lobe when an acupuncture stimulus is applied to a region adjacent to the tip of the outer from the first joint of the fourth right hand finger.

[Fig. 12]

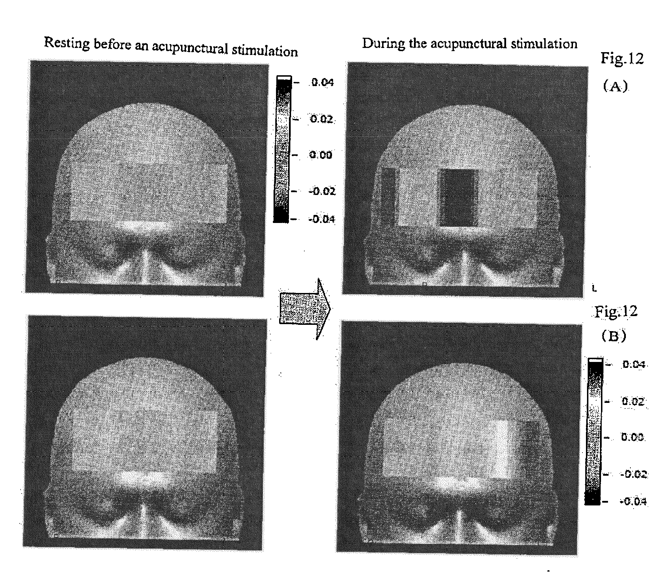

Fig. 12 (A) visually illustrates a change in the changed amount of total hemoglobin in the frontal lobe on resting before an acupuncture stimulus is applied to a region adjacent to the tip of the outer from the first joint of the fourth right hand finger and during the acupuncture.

Fig. 12 (B) visually illustrates a change in the changed amount of oxygen saturation.

[Fig. 13]

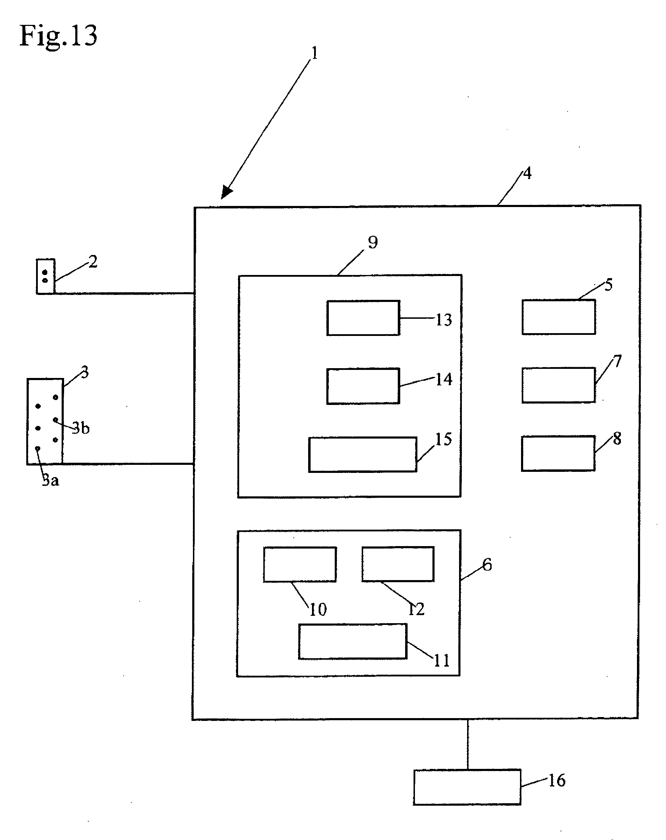

Fig. 13 is a block diagram illustrating a program according to one embodiment of the present invention.

[Best mode for carrying out the Invention]

Herein, a stimulus can comprise an electrical signal, such as that of a predetermined frequency. Herein, a stimulus applying means (or stimulus applyling device) can comprise for example at least one electrode, for example a pair of electrodes, and/or can comprise a signal generator. In particular, the stimulus applying means can comprise an electrical signal generator for generating an electrical signal of a predetermined frequency, and a body portion adapted for being applied to an acupoint of (in) the human body for stimulating the acupoint of (in) the human body. The electrical signal generator and the body portion are electrically connected e.g. by an electrical signal transmitting path. Further, a quantity of stimulus can be for example an amplitude, such as an amplitude of an electrical signal, or a time for which an amplitude is above a predetermined threshold. Herein a light detection means or light detection device can comprise for example at least one photodiode, CCD camera, and/or photomultiplier (for example as a light receiving section for sensing light); and may optionally further comprise (for example as a light emitting section) a bulb, filament, light emitting diode, and/or laser, to name several examples. A calculating means can be for example an electronic circuit, microprocessor, and/or computer or the like. A determination means can be for example an electronic circuit, microprocessor, and/or computer or the like, which may optionally serve more than one function, e.g. as a determination means and a calculating means. A stimulus adjusting means can be for example a signal generator having an adjustable amplitude. At least two of the stimulus applying means, light detection means, calculating means, determination means, and stimulus adjusting means can be operatively and/or communicatively coupled. Herein brain state and mode of the brain state may be used synonomously; for example, as used herein, a brain state in or of one of a relaxation mode, concentration mode, or intermediate mode or the like is synonymous with a mode of a brain state being in or of one of a relaxation mode (or relaxed state), concentration mode, intermediate mode, or the like. One embodiment of the present invention will now be described with reference to the drawings.

FIG.1 is a block diagram showing the arrangement of a brain state support apparatus 1 according to one embodiment of the present invention.

As can be seen from Fig. 1, the brain state support apparatus 1 according to the present invention comprises a light detecting device 3, and a stimulus applying device 2 mounted on the human body at its acupoint (e.g., a part of the left hand thumb) and configured to stimulates the acupoint with an electrical signal having a predetermined frequency, a light emitting section (light-emitting element) 3a mounted on a predetermined region of the human body (e.g., the cerebral frontal lobe) and configured to irradiate this region with light and a light receiving section (light-receiving element) 3b configured to receive and sensing light emitted from the interior of the human body, and a main apparatus body 4.

Fig. 2 (A) is a front view showing a stimulus applying device 2; Fig. 2 (B) illustrates the stimulus applying device 2 attached to a thumb; Fig. 2 (C) is a front view illustrating a stimulus applying device 2 according to another embodiment of the present invention; and Fig. 2 (D) illustrates the stimulus applying device 2 of Fig. 2 (C) attached to a finger other than the thumb.

As shown in Figs. 2 (A and B), the stimulus applying device 2 comprises a pair of stimulating portions 2a mounted on the human body in engagement with its regions to be stimulated for stimulating the regions with electrical signals of a predetermined frequency, and a band portion 2b releasably mounted on a finger for holding the stimulating portions 2a.

The pair of stimulating portions 2a may be mounted, for example, on a finger and spaced from each other with a distance between about 3 and 10 mm.

As shown in Fig. 2 (B), the pair of stimulating portions 2 are mounted on the opposite sides of a region S to be stimulated. The distance between the stimulating portions 2a may be suitably selected depending on a desired acupoint of the human body.

The band portion 2b may include loop fasteners 2c (e.g., magic tapes (registered trademark)) attached thereto on the opposite ends thereof.

The stimulus applying device 2 can be fixedly mounted on a finger by winding the band portion 2b around finger and engaging the loop fastener 2c with each other.

As shown in Figs. 2 (C) and (D), however, the width of the band portion 2b may be increased such that the pair of stimulating portions 2a may be located spaced away from each other in the diagonal direction.

The light detecting device 3 may have a harness which can be mounted on the human body at a predetermined region.

The light emitting and receiving sections 3a, 3b may be mounted on the harness and spaced apart from one another with a predetermined spacing.

The main apparatus body 4 is configured to control the operations of the stimulus applying device 2 and light detecting device 3 and to perform the input/output, computation and storage of various data.

The main apparatus body 4 comprises an input section 5, an output section 6, a communication section 7 and a storage section 8 and a control section 9.

The input section 5 is used to input various data and may be in the form of a keyboard, a numeric keypad, a mouse, a mark sheet reader or an optical character recognition (OCR) unit.

The output section 6 is used to output various data and comprises a display section 10 such as a monitor or display for displaying various data, a speaker 11 for outputting voice data and a print section 12 for printing various data.

The communication section 7 is connected to a communication network such as Internet (Data Transfer Network using Transmission Control Protocol/Internet Protocol (TCP/IP)) or Local Area Network (LAN) for transmitting and receiving various data.

For example, the communication section 7 may be in the form of a modem, a terminal adaptor, a router or Digital Service Unit (DSU).

The storage section 8 is used to store various data and comprises a database.

The control section 9 comprises a calculating section 13 for calculating the changed amount of total hemoglobin that is the sum of the changed amount of oxyhemoglobin plus the changed amount of deoxyhemoglobin and the changed amount of oxygen saturation that is a difference between the changed amount of the oxyhemoglobin and the changed amount of the deoxyhemoglobin, based on light information detected by the light detecting device 3; a determination section 14 for determining whether the brain state of the human body is in at least one of a relaxation mode, a concentration mode and an intermediate mode, based on the changed amount of total hemoglobin and the changed amount of oxygen saturation change which are calculated by the calculating section 13 by applying a stimulus of an electrical signal having a predetermined frequency to the acupoint of the human body through the stimulus applying device 2; and a stimulus adjusting section 15 for adjusting the quantity of stimulus which is applied to the acupoint of the human body with the electrical signal having a predetermined frequency by the stimulus applying means such that the mode of the brain state determined by the determination means can be maintained or shifted to the other mode of the brain state.

The determining section 14 is configured to determine that the brain state is in the relaxation mode when the changed amount of total hemoglobin is increased and also the changed amount of oxygen saturation is increased by stimulating the acupoint of the human body with an electrical signal of a first frequency (e.g., 3 Hz); to determine that the brain state is in the concentration mode when the changed amount of total hemoglobin and the changed amount of oxygen saturation are decreased by stimulating the acupoint of the human body with an electrical signal of a second frequency (e.g., 10 Hz); and to determine that the brain state is in the intermediate mode in the other cases.

The stimulus adjusting section 15 is configured to stimulate the acupoint of the human body with the electrical signal of the first frequency (e.g., 3 Hz) at (for) a predetermined interval of time when it is wanted to maintain the brain state at the relaxation mode or to shift it to the relaxation mode; and to stimulate the acupoint of the human body with the electrical signal of the second frequency (e.g., 10 Hz) at (for) a predetermined interval of time when it is wanted to maintain the brain state at the concentration mode or to shift it to the concentration mode.

The stimulus adjusting section 15 is operative to increase the quantity of stimulus when it is wanted to shift the brain state to the other mode rather than when it is wanted to maintain the brain state at the same mode.

In this regard, the frequency of the electrical signal for stimulation is not limited to the above numerical level.

Fig. 3 is a flowchart illustrating the operation of a brain state support apparatus 1 according to one embodiment of the present invention.

First of all, the stimulus applying device 2 is mounted on the human body at a region adjacent to its desired acupoint (e.g., an acupoint on the left hand thumb) (Step S1).

Subsequently, the light detecting device 3 is mounted on the human body at a predetermined region (e.g., cerebral frontal lobe) (Step S2).

Subsequently, the apparatus is prepared for determination of the brain state (Step S3).

In this Step S3, a stimulus of 3 Hz is applied to the left hand thumb for 5-10 seconds to determine the minimum strength of stimulus that is slightly felt by the skin thereof.

Further, the same stimulus of 3 Hz is applied to the left hand thumb for 5-10 seconds to determine the maximum strength of stimulus by which the skin thereof feels an ache.

Next, the intermediate (50%) strength of the 3 Hz stimulus is calculated using the following formula:

$$\text{the intermediate (50\%) strength of the 3 Hz stimulus} = (\text{the minimum strength of stimulus} + \text{the maximum strength of stimulus})/2$$

Further, a stimulus of 10 Hz is applied to the left hand thumb for 5-10 seconds to determine the minimum strength of stimulus that is slightly felt by the skin thereof.

Further, the same stimulus of 10 Hz is applied to the left hand thumb for 5-10 seconds to determine the maximum strength of stimulus by which the skin thereof feels an ache.

Next, the intermediate (50%) strength of the 10 Hz stimulus is calculated using the following formula:

$$\text{the intermediate (50\%) strength of the 10 Hz stimulus} = (\text{the minimum strength of stimulus} + \text{the maximum strength of stimulus})/2$$

The maximal strength of stimulus is set by setting the voltage and current of the electrical stimulation.

Subsequently, the brain state is determined by the determining section 14 (Step S4).

Here, the brain reaction to be provided by the stimulus of 3Hz to the left hand thumb is defined as a relaxation mode (R mode).

This mode indicates a state in which the brain is relaxed.

Further, the brain reaction to be provided by the stimulus of 10 Hz is defined as a concentration mode (B mode).

This mode indicates a state in which the brain acts to increase the concentration and intelligence. Such a state is suitable for a study, for example.

In this Step S4, the intermediate strength of stimulus of 3Hz determined in Step S2 is applied to the region of the left hand thumb in a pattern of 30 second stimulation - 30 second rest -30 second stimulation - 30 second rest.

If there is no reaction after the stimulation has been continued for 30 seconds, it is determined that the brain state is already in a stronger R mode.

If there is a reaction of R mode, it is determined that the brain state is in a lower R mode or the B mode or the intermediate mode.

Further, the intermediate strength of stimulus of 10 Hz determined in Step S2 is applied to the region of the left hand thumb in a pattern of 30 second stimulation - 30 second rest -30 second stimulation - 30 second rest.

If there is no reaction after the stimulation for 30 seconds, it is determined that the brain state is already in a stronger B mode.

If there is a reaction of B mode, it is determined that the brain state is in a lower B mode or the R mode or the intermediate mode.

The results of determination are displayed on the display section 10 such as a monitor or a display with sounds being outputted through the speaker 11.

Further, the results of determination may be printed by the printing portion 12 or transmitted as data by the communication section 7 through the network.

Subsequently, the degree of stimulation is adjusted by the stimulus adjusting section 15 (Step S5).

In this Step S5:

1) When it is judged by the determining device 14 that the brain state is in the B mode, the stimulation is adjusted such that the brain state can be shifted to the R mode.

2) When it is judged by the determining device 14 that the brain state is in the R mode, the stimulation is adjusted such that the brain state can be maintained in the R mode.

3) When it is judged by the determining device 14 that the brain state is in the B mode, the stimulation is adjusted such that the brain state can be maintained in the B mode.

4) When it is judged by the determining device 14 that the brain state is in the R mode, the stimulation is adjusted such that the brain state can be shifted to the B mode.

Either of the above cases can be selected through the input section 5.

The adjustment of each case may be practiced as follows.

1) Shift from B to R

The stimulus of 50% of the 3 Hz stimulus determined in Step S3 is applied for 60 seconds and then halted for 30 seconds. Further, the stimulus of 75% is applied for 60 seconds and halted for 30 seconds.

If the brain state is not shifted to the R mode, the 100% stimulus is further applied for 60 seconds.

In such a manner, the shift to R mode will be forced by increasing the quantity of stimulus.

2) Maintenance of R mode

The stimulus of 25% of the 3 Hz stimulus determined in Step S3 is applied for 30 seconds and then halted for 30 seconds. Further, the stimulus of 25% is applied for 30 seconds and halted for 30 seconds and applied for 30 seconds.

In such a manner, the R mode will be maintained by intermittently applying the same stimulus.

3) Maintenance of B mode

The stimulus of 25% of the 10 Hz stimulus determined in Step S3 is applied for 30 seconds and then halted for 30 seconds.

Further, the stimulus of 25% is applied for 30 seconds, then halted for 30 seconds and applied for 30 seconds.

In such a manner, the B mode will be maintained by intermittently applying the same stimulus.

4) Shift from R to B

The stimulus of 50% of the 10 Hz stimulus determined in Step S3 is applied for 60 seconds and then halted for 30 seconds. Further, the stimulus of 75% is applied for 60 seconds and then halted for 30 seconds.

If the brain state is not shifted to the B mode, the 100% stimulus is further applied for 60 seconds.

The shift to B mode will be forced by increasing the quantity of stimulus in such a manner.

In this regard, the adjustment of stimulus is not limited and can be suitably performed.

Experiments performed by the inventor will be described to prove that the brain state support apparatus 1 according to the present invention is useful.

In these experiments, the detection and record of the Hb concentration of cerebrocortical were practiced by using a near-infrared spectroscopy measuring equipment (Shimazu Corporation: FOIRE3000).

The sampling of the hemoglobin was 70 ms.

The recorded hemoglobin was oxyhemoglobin (oxy-Hb), and deoxyhemoglobin (deoxy-Hb).

The changed of total hemoglobin (total-Hb: the sum of oxy-Hb and deoxy-Hb) and the changed of oxygen saturation ($ScO_2$) are calculated to provide an index of cerebral COE reaction by the following calculating formulas:

$$\text{the changed amount of total hemoglobin (concentration)} \quad [\text{Total hemoglobin}] = [\text{Oxyhemoglobin}] + [\text{Deoxyhemoglobin}] \text{------- formula (1)}$$

The changed amount of oxygen saturation (concentration) (a brain function index which was found by Kato, one of the inventors)

$$[\text{ScO2}] \text{ (oxygen saturation or oxygen exchange)} = [\text{Oxyhemoglobin}] - [\text{Deoxyhernoglobin}] \text{------ formula (2)}$$

Here, [Oxyhemoglobin] is the changed amount of oxyhemoglobin (concentration). [Deoxyhernoglobin] is the changed amount of deoxyhemoglobin (concentration).

A particularly important function index is a decrease of $ScO_2$ = oxygen consumption.

An oxygenated region with reduced concentration of oxygen is particularly important.

The changed amount of total hemoglobin represents variations in the number of red blood cells in a voxel of an optical measurement region which is sandwiched between irradiation and detection probes.

If the changed amount of total hemoglobin increases, it means the increased number of red blood cells.

If the changed amount of total hemoglobin decreases, it means the decreased number of red blood cells.

The changed amount of oxygen saturation (oxygen exchange) indicates the changed concentration of oxygen in the capillary.

If the changed amount of oxygen saturation increases, it means the increased amount of oxygen in the blood vessel.

If the changed amount of oxygen saturation decreases, it means the decreased amount of oxygen in the blood vessel.

The increased amount of oxygen in the blood vessel means the delivery of red blood cells rich in oxygen.

The decreased amount of oxygen in the blood vessel means that oxygen in the capillary has been consumed by the nerve cells.

The results of experiments are indicated using two kinds of waveform indication and mapping indication.

The waveform indication shows time series variations while the mapping indication is a plot of integrated values for a predetermined period of task time (stimulus time).

Figs. 4 (A) - (C) illustrate the arrangement of the light detecting device 3.

The light detecting device 3 is disposed in an arrangement of high-density probe to cover the Brodmann's tenth area (frontal lobe) shown in Fig. 4 (A).

The light emitting portions 3a and light receiving portions 3b of the light detecting device 3 are disposed spaced apart from each other by such a distance as shown in Fig. 4 (B).

As can be seen from Fig. 4 (C), three light emitting portions 3a and three light receiving portions 3b are placed between the right and left brain regions.

Changes of the changed amount of total hemoglobin and changes of the changed amount of oxygen saturation are measured at five measurement points of 24ch-28ch in total.

Fig. 5 shows graphs showing changes in the changed amount of total hemoglobin and the changed amount of oxygen saturation in the frontal lobe when an electrical stimulus of 3Hz is applied to a region adjacent to the center of the side of the hand palm between the first and second joints of the left hand thumb.

Here, the horizontal axis indicates time (s) while the vertical axis indicates the amount of change (mol/l). The time of stimulation is 30 seconds.

Fig. 6 (A) visually illustrates a change in the changed amount of total hemoglobin in the frontal lobe on resting before an electrical stimulus of 3Hz is applied to a region adjacent to the center of the side of the hand palm between the first and second joints of the left hand thumb and on stimulation after such an electrical stimulus has been applied to the same region.

Fig. 6 (B) visually illustrates a change in the changed amount of oxygen saturation.

As can be seen from Figs. 5 and 6, it was observed that dynamic increases of the changed amount of total hemoglobin and the changed amount of oxygen saturation over a widened range were derived by the electrical stimulation of 3 Hz. Particularly, these changes were conspicuously recognized in the frontal lobe.

Fig. 7 shows graphs showing changes in the changed amount of total hemoglobin and the changed amount of oxygen saturation in the frontal lobe when an electrical stimulus of 10 Hz is applied to a region adjacent to the center of the side of the hand palm between the first and second joints of the left hand thumb.

Here, the horizontal axis indicates time (s) while the vertical axis indicates the amount of change (mol/l). The time of stimulation is 30 seconds.

Fig. 8 (A) visually illustrates a change of the changed amount of total hemoglobin in the frontal lobe on resting before an electrical stimulus of 10Hz is applied to a region adjacent to the center of the side of the hand palm between the first and second joints of the left hand thumb and on stimulation after such an electrical stimulus has been applied to the same region.

Fig. 8 (B) visually illustrates a change in the changed amount of oxygen saturation.

As can be seen from Figs. 7 and 8, it was observed that the changed amount of total hemoglobin and the changed amount of oxygen saturation were dynamically decreased by the electrical stimulation of 10 Hz over a widened range.

When the electrical signals of 3 Hz and 10 Hz are applied to the targeted region of the left hand thumb in such a manner, respectively, the oxygen metabolism in the frontal lobe was opposite.

With the stimulation of 10 Hz, the oxygenation is reduced.

With the stimulation of 3 Hz, the bloodstream and oxygen were increased.

The state of the frontal lobe was sensitively changed depending on the frequency of the electrical stimulation.

This suggests that the cognitive function is affected by the electrical stimulation.

Next, experiments when an acupunctural stimulation was performed as reference to compare it with the present invention will be described.

Fig. 9 shows graphs showing changes in the changed amount of total hemoglobin and the changed amount of oxygen saturation in the frontal lobe when an acupuncture stimulus is applied to a region adjacent to the center of the side of the hand palm between the first and second joints of the left hand thumb.

Here, the horizontal axis indicates time (s) while the vertical axis indicates the amount of change (mol/l). The time of stimulation is 180 seconds.

Fig. 10 (A) visually illustrates changes in the changed amount of total hemoglobin in the frontal lobe on resting when an acupunctural stimulation is applied to a region adjacent to the center of the side of the hand palm between the first and second joints of the left hand thumb, during the acupunctural stimulation and after the extraction of the acupunctural stimulation.

Fig. 10 (B) visually illustrates a change in the changed amount of oxygen saturation.

As can be seen from Figs. 9 and 10, the acupunctural stimulation increased both the changed amount of total hemoglobin and the changed amount of oxygen saturation for 30-45 seconds on start of the acupuncture rotation.

However, the changed amount of total hemoglobin and changed amount of oxygen saturation were thereafter decreased continuously.

It is conceivable that this is the main effect of the acupuncture.

Further, the repeatability was recognized with respect to that the stimulation of 10 Hz is more similar to the acupunctural stimulation than the stimulation of 3 Hz.

Fig. 11 shows graphs illustrating changes in the changed amount of total hemoglobin and the changed amount of oxygen saturation in the frontal lobe when an acupuncture stimulus is applied to a region adjacent to the tip of the outer from the first joint of the fourth right hand finger.

Here, the horizontal axis indicates time (s) while the vertical axis indicates the amount of change (mol/l). The time of stimulation is 180 seconds.

Fig. 12 (A) visually illustrates a change in the changed amount of total hemoglobin in the frontal lobe on resting before an acupuncture stimulus is applied to a region adjacent to the tip of the outer from the first joint of the fourth right hand finger and during the acupunctural stimulation.

Fig. 12 (B) visually illustrates a change in the changed amount of oxygen saturation.

As can be seen from Figs. 11 and 12, the increase of oxygenation and the decrease of bloodstream were observed in a part of the frontal lobe as a pain is being increased by the rotational acupunctural stimulations.

However, this was clearly different from the brain reaction due to the stimulation to the left hand thumb.

Fig.13 is a block diagram showing a program according to one embodiment of the present invention.

As shown in Fig.13, the program 16 according to the embodiment of the present invention is configured to control the main apparatus body 4 of the brain state support apparatus 1 according to the embodiment of the present invention and to cause a computer to perform the process in the main apparatus body 4.

This program 16 may be recorded in a recording media such as magnetic disk, CD-ROM, semiconductor memory and may be downloaded through a communication network.

The present invention is not limited to the above mentioned embodiments, but can be modified to various other forms without departing from the range of technical features described in the subsequent claims.

For example, the main apparatus body 4 may be in the form of a personal computer or information terminal equipment.

[Industrial Applicability]

The brain state support apparatus 1 and program 16 according to the present invention can be used to support the brain state such that the brain state can be maintained in a relaxation or concentration mode or shifted to the other state, using the near-infrared spectroscopy (NIRS).

[Explanation of Reference Numerals]

1: Brain State Support Apparatus
2: Stimulus applying device
2a: Stimulating Section
2b: Band Portion
2c: Loop Fasteners
3: Light detecting device
3a: Light Emitting Portions
3b: Light Receiving Portions
4: Main Apparatus Body

5: Input Section
6: Output Section
7: Communication Section
8: Storage Section
9: Control Section
10: Display Section
11: Speaker
12: Printing Section
13: Calculating Section
14: Determining Section
15: Stimulus Adjusting Section
16: Program

**Claims**

1. A brain state support apparatus (1) comprising:

stimulus applying means (2) for stimulating an acupoint in the human body with an electrical signal of a predetermined frequency;
light detection means (3) comprising a light emitting section for irradiating the human body with a light at a predetermined region, and a light receiving section for receiving and sensing a light emitted from the interior of the human body; and
a main apparatus body (4) for controlling the stimulus applying means and the light detection means,
said main apparatus body comprising:

calculating means (13) for calculating a changed amount of total hemoglobin that is the sum of a changed amount of oxyhemoglobin plus a changed amount of deoxyhemoglobin and a changed amount of oxygen saturation that is a difference between the changed amount of the oxyhemoglobin and the changed amount of the deoxyhemoglobin, based on light information detected by the light detecting means before and after stimulating the acupoint;
determination means (14) for determining whether the brain state of the human body is in at least one of a relaxation mode, a concentration mode and an intermediate mode, based on the changed amount of total hemoglobin and the changed amount of oxygen saturation as calculated by the calculating means; and
stimulus adjusting means (15) for adjusting the quantity of stimulus which is applied to the acupoint of the human body with the electrical signal having a predetermined frequency by the stimulus applying means such as to selectively maintain the mode of the brain state determined by the determination means (14) or shift it to another one of the modes of the brain state, wherein the stimulus adjusting means (15) is configured to adjust the stimulus to stimulate the acupoint of the human body with the electrical signal of a first frequency for a predetermined interval of time to maintain the mode of the brain state in the relaxation mode or to shift to the relaxation mode; and to stimulate the acupoint of the human body with the electrical signal of a second frequency for a predetermined interval of time to maintain the brain state in the concentration mode or to shift to the concentration mode.

2. The brain state support apparatus as claimed in claim 1, wherein the acupoint of the human body stimulated by the stimulus applying means is in the region of left thumb.

3. The brain state support apparatus as claimed in claim 1 or 2, wherein the region detected by the light detecting means is the cerebral frontal lobe.

4. The brain state support apparatus according to any one of claims 1-3, wherein the determination means is configured to determine that the brain state is in the relaxation mode when the changed amount of total hemoglobin is increased and also the changed amount of oxygen saturation is increased by stimulating the acupoint of the human body with an electrical signal of a first frequency; to determine that the brain state is in the concentration mode when the changed amount of total hemoglobin is decreased and also the changed amount of oxygen saturation is decreased by stimulating the acupoint of the human body with an electrical signal of a second frequency; and to determine that the brain state is in the intermediate mode in the other cases.

**5.** The brain state support apparatus as claimed in claim 4, wherein the first frequency is 3Hz and the second frequency is 10Hz.

**6.** The brain state support apparatus according to any one of claims 1-3, wherein the determination means is configured to determine that the mode of the brain state is in: the relaxation mode if the changed amount of total hemoglobin is increased and/or the changed amount of oxygen saturation is increased by stimulating the acupoint of the human body with an electrical signal of a first frequency; the concentration mode if the changed amount of total hemoglobin is decreased and and/or the changed amount of oxygen saturation is decreased by stimulating the acupoint of the human body with an electrical signal of a second frequency; and otherwise the intermediate mode.

**7.** The brain state support apparatus according to any one of claims 1-6, wherein the stimulus adjusting means is configured to increase the quantity of stimulus when the brain state is to be shifted to the other modes rather than when the brain state is to be maintained at the same mode.

**8.** A program **characterized by** that it causes a brain state support apparatus according to any one of claims 1-7 to perform the following steps:

   stimulating an acupoint with an electrical signal;
   irradiating a predetermined region of a human body with a light;
   sensing light from the interior of the human body;
   calculating a changed amount of total hemoglobin that is the sum of a changed amount of oxyhemoglobin plus a changed amount of deoxyhemoglobin based on the sensed light;
   calculating a changed amount of oxygen saturation that is a difference between the changed amount of the oxyhemoglobin and the changed amount of the deoxyhemoglobin, based on the sensed light;
   determining a mode of a brain state, wherein the mode is at least one of: a relaxation mode, a concentration mode and an intermediate mode; wherein the determination is based on the changed amount of total hemoglobin and the changed amount of oxygen saturation change;
   and adjusting the quantity of stimulus in dependence of the determined mode of the brain state, when loaded into said apparatus.

**9.** The program according to claim 8, the processing further comprising at least one of:

   stimulating the acupoint with the electrical signal of a first frequency for a predetermined interval of time to maintain and/or induce the relaxation mode;
   and stimulating the acupoint with the electrical signal of a second frequency for a second predetermined interval of time to maintain and/or induce the concentration mode.

**10.** The program according to claim 8 or 9, wherein
adjusting the quantity of stimulus comprises increasing the stimulus to induce a change of brain state; wherein the quantity is increased in comparison to a maintaining of the brain state.

**11.** A computer readable medium containing the program according to any one of claims 8 to 10.

**Patentansprüche**

**1.** Gehirnzustand-Unterstützungsvorrichtung (1), umfassend:

   Stimulus-Anwendungs-Hilfsmittel (2) zur Stimulierung eines Akupunkturpunkts im menschlichen Körper mit einem elektrischen Signal einer vorgegebenen Frequenz;
   Lichterfassungsmittel (3), umfassend einen Lichtemissionsbereich zum Bestrahlen des menschlichen Körpers mit Licht an einem vorgegebenen Bereich und einen Lichtempfangsbereich zum Empfangen und Erfassen von Licht, das aus dem Inneren des menschlichen Körpers emittiert wird; und
   einen Hauptvorrichtungskörper (4) zum Steuern und/oder Regeln des Stimulus-Anwendungs-Hilfsmittels und des Lichterfassungsmittels,
   wobei der Hauptvorrichtungskörper umfasst:

      Berechnungsmittel (13) zum Berechnen eines veränderten Betrags des Gesamt-Hämoglobins, der die Summe eines veränderten Betrags des Oxyhämoglobins plus einen veränderten Betrag des Desoxyhämoglobins ist, und eines veränderten Betrag der Sauerstoffsättigung, der eine Differenz zwischen dem veränderten Betrag des Oxyhämoglobins und dem veränderten Betrag des Desoxyhämoglobins ist, anhand von durch das Lichterfassungsmittel erfasster Lichtinformation vor und nach dem Stimulieren des Akupunkturpunkts;

Bestimmungsmittel (14) zur Bestimmung, ob sich der Gehirnzustand des menschlichen Körpers in mindestens einem der folgenden befindet: ein Entspannungsmodus, ein Konzentrationsmodus und ein Zwischenmodus, anhand des veränderten Betrags des Gesamt-Hämoglobins und des veränderten Betrags der Sauerstoffsättigung, wie von dem Berechnungsmittel berechnet; und

Stimulus-Einstellmittel (15) zum Einstellen des Umfangs des Stimulus, der an dem Akupunkturpunkt des menschlichen Körpers angewendet wird, mit dem elektrischen Signal, das eine vorgegebene Frequenz aufweist, durch das Stimulus-Anwendungs-Hilfsmittel derart, dass der durch das Bestimmungsmittel bestimmte Modus des Gehirnzustands selektiv beibehalten wird oder zu einem anderen der Modi des Gehirnzustands gewechselt wird, wobei

das Stimulus-Einstellmittel (15) so konfiguriert ist, dass es den Stimulus einstellt, so dass dieser den Akupunkturpunkt des menschlichen Körpers mit dem elektrischen Signal einer ersten Frequenz für ein vorgegebenes Zeitintervall stimuliert, um den Modus des Gehirnzustands im Entspannungsmodus beizubehalten oder in den Entspannungsmodus zu wechseln; und den Akupunkturpunkt des menschlichen Körpers mit dem elektrischen Signal einer zweiten Frequenz für ein vorgegebenes Zeitintervall stimuliert, um den Gehirnzustand im Konzentrationsmodus beizubehalten oder in den Konzentrationsmodus zu wechseln.

2. Gehirnzustand-Unterstützungsvorrichtung nach Anspruch 1, wobei der durch das Stimulus-Anwendungs-Hilfsmittel stimulierte Akupunkturpunkt des menschlichen Körpers im Bereich des linken Daumens ist.

3. Gehirnzustand-Unterstützungsvorrichtung nach Anspruch 1 oder 2, wobei der von dem Lichterfassungsmittel erfasste Bereich der zerebrale Frontallappen ist.

4. Gehirnzustand-Unterstützungsvorrichtung nach einem der Ansprüche 1-3, wobei das Bestimmungsmittel konfiguriert ist, zu bestimmen, dass der Gehirnzustand im Entspannungsmodus ist, wenn der veränderte Betrag des Gesamthämoglobins erhöht ist und auch wenn der veränderte Betrag der Sauerstoffsättigung erhöht ist durch Stimulierung des Akupunkturpunkts des menschlichen Körpers mit einem elektrischen Signal einer ersten Frequenz; zu bestimmen, dass der Gehirnzustand im Konzentrationsmodus ist, wenn der veränderte Betrag des Gesamthämoglobins verringert ist und auch wenn der veränderte Betrag der Sauerstoffsättigung verringert ist durch Stimulierung des Akupunkturpunkts des menschlichen Körpers mit einem elektrischen Signal einer zweiten Frequenz; und zu bestimmen, dass der Gehirnzustand im Zwischenmodus in den anderen Fällen ist.

5. Gehirnzustand-Unterstützungsvorrichtung nach Anspruch 4 beansprucht, wobei die erste Frequenz 3 Hz und die zweite Frequenz 10 Hz beträgt.

6. Gehirnzustand-Unterstützungsvorrichtung nach einem der Ansprüche 1-3, wobei das Bestimmungsmittel konfiguriert ist, zu bestimmen, dass der Modus des Gehirnzustands in den folgenden Zuständen ist: im Entspannungsmodus, wenn der veränderte Betrag des Gesamthämoglobins erhöht ist und/oder der veränderte Betrag der Sauerstoffsättigung erhöht ist durch Stimulierung des Akupunkturpunkts des menschlichen Körpers mit einem elektrischen Signal einer ersten Frequenz; im Konzentrationsmodus, wenn der veränderte Betrag des Gesamthämoglobins verringert ist und/oder der veränderte Betrag der Sauerstoffsättigung verringert ist durch Stimulierung des Akupunkturpunkts des menschlichen Körpers mit einem elektrischen Signal einer zweiten Frequenz; und ansonsten im Zwischenmodus.

7. Gehirnzustand-Unterstützungsvorrichtung nach einem der Ansprüche 1-6, wobei das Stimulus-Einstellmittel konfiguriert ist, den Umfang des Stimulus zu erhöhen, wenn der Gehirnzustand in die anderen Modi gewechselt werden soll anstatt, wenn der Gehirnzustand im gleichen Modus beibehalten werden soll.

8. Programm, **dadurch gekennzeichnet, dass** es eine Gehirnzustand-Unterstützungsvorrichtung nach einem der Ansprüche 1-7 veranlasst, die folgenden Schritte auszuführen:

Stimulieren eines Akupunkturpunkt mit einem elektrischen Signal;
Bestrahlen eines vorgegebenen Bereichs eines menschlichen Körpers mit Licht;
Erfassen von Licht aus dem Inneren des menschlichen Körpers;

Berechnen eines veränderten Betrags des Gesamthämoglobins, der die Summe eines veränderten Betrags des Oxyhämoglobins plus einen veränderten Betrag des Desoxyhämoglobins ist, anhand des erfassten Lichts;
Berechnen eines veränderten Betrags der Sauerstoffsättigung, der eine Differenz zwischen dem veränderten Betrag des Oxyhämoglobins und dem veränderten Betrag des Desoxyhämoglobins ist, anhand des erfassten Lichts;
Bestimmen eines Modus eines Gehirnzustands, wobei der Modus zumindest einer der folgenden ist: ein Entspannungsmodus, ein Konzentrationsmodus und ein Zwischenmodus; wobei das Bestimmen auf dem veränderten Betrag des Gesamt-Hämoglobins und dem veränderten Betrag der Sauerstoffsättigung beruht;
und Einstellen des Umfangs des Stimulus in Abhängigkeit von dem bestimmten Modus des Gehirnzustands, wenn es in die Vorrichtung geladen wird.

9. Programm nach Anspruch 8, wobei die Verarbeitung ferner mindestens eines der folgenden umfasst:

Stimulierung des Akupunkturpunkts mit dem elektrischen Signal einer ersten Frequenz für ein vorgegebenes Zeitintervall, um den Entspannungsmodus beizubehalten und/oder zu veranlassen;
und Stimulierung des Akupunkturpunkts mit dem elektrischen Signal einer zweiten Frequenz für ein vorgegebenes Zeitintervall, um den Konzentrationsmodus beizubehalten und/oder zu veranlassen.

10. Programm nach Anspruch 8 oder 9, wobei das Einstellen des Umfangs des Stimulus die Erhöhung des Stimulus umfasst, um eine Änderung des Gehirnzustands zu veranlassen; wobei der Umfang im Vergleich zu einer Beibehaltung des Gehirnzustands erhöht wird.

11. Computerlesbares Medium, das das Programm nach einem der Ansprüche 8 bis 10 umfasst.

## Revendications

1. Appareil de support d'état cérébral (1) comprenant :

des moyens d'application de stimulus (2) pour stimuler un point d'acupuncture dans le corps humain avec un signal électrique d'une fréquence prédéterminée ;
des moyens de détection de lumière (3) comprenant une section émettrice de lumière pour irradier le corps humain avec une lumière au niveau d'une région prédéterminée, et une section réceptrice de lumière pour recevoir et détecter une lumière émise depuis l'intérieur du corps humain : et
un corps d'appareil principal (4) pour contrôler les moyens d'application de stimulus et les moyens de détection de lumière,
ledit corps d'appareil principal comprenant :

des moyens de calcul (13) pour calculer une quantité changée d'hémoglobine totale qui est la somme d'une quantité changée d'oxyhémoglobine plus une quantité changée de déoxyhémoglobine et une quantité changée de saturation en oxygène qui est une différence entre la quantité changée de l'oxyhémoglobine et la quantité changée de la déoxyhémoglobine, sur la base d'informations de lumière détectées par les moyens de détection de lumière avant et après la stimulation du point d'acupuncture,
des moyens de détermination (14) pour déterminer si l'état cérébral du corps humain est dans au moins l'un d'un mode de relaxation, d'un mode de concentration et d'un mode intermédiaire, sur la base de la quantité changée d'hémoglobine totale et de la quantité changée de saturation en oxygène telle que calculée par les moyens de calcul ; et
des moyens d'ajustement de stimulus (15) pour ajuster la quantité de stimulus qui est appliquée au point d'acupuncture du corps humain avec le signal électrique ayant une fréquence prédéterminée par les moyens d'application de stimulus de telle sorte à maintenir sélectivement le mode de l'état cérébral déterminé par les moyens de détermination (14) ou à le faire passer à un autre des modes de l'état cérébral, dans lequel les moyens d'ajustement de stimulus (15) sont configurés pour ajuster le stimulus pour stimuler le point d'acupuncture du corps humain avec le signal électrique d'une première fréquence pendant un intervalle de temps prédéterminé pour maintenir le mode de l'état cérébral dans le mode de relaxation ou le faire passer en mode de relaxation ; et pour stimuler le point d'acupuncture du corps humain avec le signal électrique d'une seconde fréquence pendant un intervalle de temps prédéterminé pour maintenir l'état cérébral dans le mode de concentration ou le faire passer en mode de concentration.

**EP 2 606 931 B1**

**2.** Appareil de support d'état cérébral selon la revendication 1, dans lequel le point d'acupuncture du corps humain stimulé par les moyens d'application de stimulus est dans une région du pouce gauche.

**3.** Appareil de support d'état cérébral selon la revendication 1 ou 2, dans lequel la région détectée par les moyens de détection de lumière est le lobe frontal cérébral.

**4.** Appareil de support d'état cérébral selon l'une quelconque des revendications 1 à 3, dans lequel les moyens de détermination sont configurés pour déterminer que l'état cérébral est dans le mode de relaxation lorsque la quantité changée d'hémoglobine totale est augmentée et également que la quantité changée de saturation en oxygène est augmentée par stimulation du point d'acupuncture du corps humain avec un signal électrique d'une première fréquence ; pour déterminer que l'état cérébral est dans le mode de concentration lorsque la quantité changée d'hémoglobine totale est réduite et également que la quantité changée de saturation en oxygène est réduite par stimulation du point d'acupuncture du corps humain avec un signal électrique d'une seconde fréquence, et pour déterminer que l'état cérébral est dans le mode intermédiaire dans les autres cas.

**5.** Appareil de support d'état cérébral selon la revendication 4, dans lequel la première fréquence est 3 Hz et la seconde fréquence est 10 Hz.

**6.** Appareil de support d'état cérébral selon l'une quelconque des revendications 1 à 3, dans lequel les moyens de détermination sont configurés pour déterminer que le mode de l'état cérébral est : dans le mode de relaxation si la quantité changée d'hémoglobine totale est augmentée et/ou la quantité changée de saturation en oxygène est augmentée en stimulant le point d'acupuncture du corps humain avec un signal électrique d'une première fréquence ; dans le mode de concentration si la quantité changée d'hémoglobine totale est réduite et/ou la quantité changée de saturation en oxygène est réduite en stimulant le point d'acupuncture du corps humain avec un signal électrique d'une seconde fréquence ; et sinon dans le mode intermédiaire.

**7.** Appareil de support d'état cérébral selon l'une quelconque des revendications 1 à 6, dans lequel les moyens d'ajustement de stimulus sont configurés pour augmenter la quantité de stimulus lorsque l'état cérébral doit être passé à d'autres modes plutôt que lorsque l'état cérébral doit être maintenu au même mode.

**8.** Programme **caractérisé en ce qu'**il amène un appareil de support d'état cérébral selon l'une quelconque des revendications 1 à 7 à effectuer les étapes suivantes :

la stimulation d'un point d'acupuncture avec un signal électrique ;
l'irradiation d'une région prédéterminée d'un corps humain avec une lumière ;
la détection de la lumière provenant de l'intérieur du corps humain ;
le calcul d'une quantité changée d'hémoglobine totale qui est la somme d'une quantité changée d'oxyhémoglobine plus une quantité changée de déoxyhémoglobine sur la base de la lumière détectée ;
le calcul d'une quantité changée de saturation en oxygène qui est une différence entre la quantité changée de l'oxyhémoglobine et la quantité changée de la déoxyhémoglobine, sur la base de la lumière détectée ;
la détermination d'un mode d'état cérébral, le mode étant au moins l'un parmi : un mode de relaxation, un mode de concentration et un mode intermédiaire ; la détermination étant basée sur la quantité changée d'hémoglobine totale et la quantité changée de saturation en oxygène ;
et l'ajustement de la quantité de stimulus en fonction du mode déterminé de l'état cérébral,
lorsqu'il est chargé dans ledit appareil.

**9.** Programme selon la revendication 8, le traitement comprenant en outre au moins l'une parmi :

la stimulation du point d'acupuncture avec le signal électrique d'une première fréquence pendant un intervalle de temps prédéterminé pour maintenir et/ou induire le mode de relaxation ;
et la stimulation du point d'acupuncture avec le signal électrique d'une seconde fréquence pendant un second intervalle de temps prédéterminé pour maintenir et/ou induire le mode de concentration.

**10.** Programme selon la revendication 8 ou 9, dans lequel
l'ajustement de la quantité de stimulus comprend l'augmentation du stimulus pour induire un changement d'état cérébral, dans lequel la quantité est augmentée par rapport à un maintien de l'état cérébral.

**11.** Support lisible par ordinateur contenant le programme selon l'une quelconque des revendications 8 à 10.

Fig.1

# Fig.2

(A)

(C)

(B)

(D)

# Fig.3

```
        ( start )
           |
   +-----------------+
   |  the device 2 is |——— S1
   |     mounted      |
   +-----------------+
           |
   +-----------------+
   |  the device 3 is |——— S2
   |     mounted      |
   +-----------------+
           |
   +-----------------+
   |   preparation    |——— S3
   +-----------------+
           |
   +-----------------+
   |  determination   |——— S4
   +-----------------+
           |
   +---------------------+
   | adjustment of stimulus |——— S5
   +---------------------+
           |
        (  end  )
```

Fig.4 (A)

Fig.4 (B)

Fig.4 (C)

Fig.5

Resting before an electrical stimulus of 3Hz

During an electrical stimulus of 3Hz

Fig.6 (A)

Fig.6 (B)

Fig.7

Resting before an electrical stimulus of 10Hz

During an electrical stimulus of 10Hz

Fig.8
(A)

— 0.04

— 0.02

— 0.00

— -0.02

— -0.04

Fig.8
(B)

— 0.04

— 0.02

— 0.00

— -0.02

— -0.04

Fig.9

Resting before an acupunctural stimulation

During the acupunctural stimulation

After the extraction of the acupunctural stimulation

Fig.10 (A)

Fig.10 (B)

Fig.11

Resting before an acupunctural stimulation

During the acupunctural stimulation

Fig.12
(A)

Fig.12
(B)

Fig.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002177282 A
- US 2007167690 A1
- US 2009228084 A1

**Non-patent literature cited in the description**

- **LITSCHER GERHARD.** Bioengineering assessment of acupuncture, part 5: cerebral near-infrared spectroscopy. *CRITICAL REVIEWS IN BIOMEDICAL ENGINEERING,* 01 January 2006, vol. 34 (6), 439-457
- **AKIKAZU SAKUDO et al.** Visible and near-infrared spectral changes in the thumb of patients with chronic fatigue syndrome. *CLINICA CHIMICA ACTA,* 01 May 2009, vol. 403 (1-2), 163-166